# EUROPEAN PATENT APPLICATION

(11) **EP 2 808 026 A1**
(43) Date of publication of application: **03.12.2014**
(21) Application number: 14001905.0
(22) Date of filing: 02.06.2014
(51) Int. Cl.: A61K 33/06, A61K 31/355, A61K 31/4415, A61K 31/202, A61P 15/12

(54) **Composition for treatment or reduction of symptoms related to premenstrual syndrome (pms), premenstrual disphoric disorder (pmdd), premenopause, menopause or female hormonal disorders in a pharmaceutical composition containing said form, and process for producing said pharmaceutical form and use of said composition**

(30) Priority: 31.05.2013 BR 102013013564
(71) Applicant: EMS S.A., CEP-13186-901 Hortolandia, SP (BR)
(72) Inventor: Barufaldi, Thalita Plassa, São Paulo (BR); Masiero, Silvana, São Paulo (BR); Nadaluti, Rebeca Magalhães, São Paulo (BR); Khater Covesi, Leticia, São Paulo (BR); Vilar, Wladia Möller, São Paulo (BR)
(74) Representative: Ricker, Mathias

(57) **Abstract**

The present invention relates to the field of food and medicines for the treatment or alleviation of premenstrual syndrome (PMS), premenstrual dysphoric disorder (PMDD), premenopause, menopause and female hormonal disorders. A first embodiment relates to a composition containing vitamins, minerals and oils. Additionally, the invention comprises a pharmaceutical form, more specifically in the form of soft capsules (also known as gelatin) containing the composition of the invention. In particular, the compositions of the invention contain vitamins and minerals in sources compatible with the association of vegetable oils, preferably borage oil, evening primrose oil, or a mixture of two oils, wherein the soft capsule contains a liquid and oily core.

## Description

### Field of the Invention

The present invention relates to the field of food and medicines for the treatment or alleviation of premenstrual syndrome (PMS), premenstrual dysphoric disorder (PMDD), premenopause, menopause and female hormonal disorders. A first embodiment relates to a composition containing vitamins, minerals and oils. Additionally, the invention comprises a pharmaceutical form, more specifically in the form of soft capsules (also known as gelatin) containing the inventive composition. In particular, the compositions of the invention contain vitamin vitamins and mineral minerals presents present in compatible sources for association with a vegetable oil, preferably borage oil, evening primrose oil, or a mixture of both oils, wherein the soft capsule contains a liquid and oily core.

### Background of the Invention

Premenstrual syndrome (PMS) or premenstrual tension (PMT) is represented by a set of physical, emotional and behavioral symptoms that present cyclical and recurrent character, beginning one to two weeks before menstruation begins, that is, during the luteal phase, and ease with the onset of menses.

These symptoms are typically severe enough to interfere with some aspects of life. Mild symptoms during the premenstrual period reported by most women and that do not interfere with daily routine are not considered for the diagnosis of PMS.

The pathophysiology of PMS is still unclear and remains the subject of study. Current treatments for PMS and other hormonal disorders include oral contraceptives, agonists of gonadotropin-releasing hormone and antidepressants, which have significant side effects (Panay N, Studd JW. The psychotherapeutic effects of estrogens. Endocrinol Gynecol 1998; 12:353-65).

Premenstrual syndrome and premenstrual dysphoric disorder (PMDD) are two symptoms related to premenstrual phase disorders. Premenstrual dysphoric disorder is considered as a subtype as well as the most severe form of premenstrual syndrome (Marvan ML, Cortes-Iniestra S. Women's beliefs about the prevalence of premenstrual syndrome and biases in recall of premenstrual changes. Health Psychol 2001; 20: 276-80). The prevalence of symptoms of premenstrual syndrome is 75% to 80% in women of reproductive age and with great variation in the number, duration and severity of symptoms. The prevalence of premenstrual dysphoric disorder is from 3% to 8% with the symptoms being related to mood, as in a deficit of social, work and family functioning. (Sadler C, Smith M, Hammond J, Bayly R, Borland S, Panay N, et al. Lifestyle factors, hormonal contraception, and premenstrual symptoms: the United Kingdom Southampton Women's Survey. J Women's Health (Larchmt) 2010, 19:391-6; Freeman EW. Premenstrual syndrome and premenstrual dysphoric disorder: definitions and diagnosis. Psychoneuroendocrinology 2003, 28 Suppl 3:25-37; Rapkin AJ, JA Mikacich. Premenstrual syndrome and premenstrual dysphoric disorder in adolescents. Curr Opin Obstet Gynecol 2008; 20:455-63; Halbreich U, Borenstein J, Pearlstein T, Kahn LS. The prevalence, impairment, impact, and burden of premenstrual dysphoric disorder (PMS / PMDD). Psychoneuroendocrinology 2003; 28 Suppl 3: 1-23). In Brazil, studies in ambulatory gynecology demonstrate the prevalence of premenstrual syndrome to be between 8% and 86%, depending on the intensity of the symptoms. Among reported symptoms, 86% were related to irritability, 17% fatigue, 62% depression and headache (each) with 95% of women having more than one symptom and 76% associated with physical and psychological symptoms (Diegoli MSC, Fonseca AM, Diegoli CA, HW Halbe, Bagnoli VR, Pinotti JA Premenstrual syndrome: a study of the incidence and symptomatic variations. Rev Obstet Gynecol 1994; 5:238-42). A Brazilian population-based study found a prevalence of premenstrual syndrome of 95.4%, considering one symptom as diagnostic, and 25.2% considering five symptoms which interfered in family or social life (CML Silva, Gigante DP, Carret MLV Fassa AG. Population study of premenstrual syndrome. Rev Public Health 2006; 40:47-56).

The signs and symptoms are physical and psychological or behavioral, presenting a temporal relationship with the premenstrual period and with features repeating in each new cycle. Signs and symptoms which are usually observed are: increased size and sensitivity of the breasts; swelling in the legs and sometimes the whole body; weight gain; headache; fatigue; leg pain; increased abdominal size; acne; anxiety; irritability; depression; and changes in mood and appetite. The intensity and quality of the signs and symptoms are quite variable from woman to woman, and have been reported from menarche to menopause. Most women seek help for PMS by the age of 30, after 10 years or more of symptoms (Halbreich U, Borenstein J, Pearlstein T, Kahn LS. The prevalence, impairment, impact, and burden of premenstrual dysphoric disorder (PMS / PMDD).Psychoneuroendocrinology 2003, 28 Suppl 3:1-23).

To determine the treatment for premenstrual syndrome or premenstrual tension, some basic principles must be observed. Because the symptoms of PMS and PMDD are chronic and recurring, the treatment should first take into account the cost of adverse effects. Secondly, treatment must be adapted to the severity of symptoms, with the selection of medicines and other regimes aligned with the needs of the patient. Therapeutic interventions for PMS range from a conservative approach (no drug treatment) to the completion of medical or even surgical treatment (Rapkin AJ, JA Mikacich. Premenstrual syndrome and premenstrual dysphoric disorder in adolescents. Curr Opin Obstet Gynecol 2008; 20:455-6; Bethea CL. Regulation of progestin receptors in raphe neurons of steroid treated monkeys. Neuroendocrinology 1994; 60:50-61). Among the proposed non-pharmacological interventions, are changes in lifestyle, including the practice of aerobic exercise, changes in diet and use of herbal preparations. Many nutritional behaviors have been discussed and studied in order to minimize the symptoms of PMS.

Vitamin B6 deficiency in patients with PMS has been considered one of the causes, since this vitamin acts as a coenzyme in the biosynthesis of dopamine and serotonin (neurotransmitters possibly involved in the etiology of PMS). It is also noted that its administration correlates with an increase in serum progesterone in the mid-luteal period, with an influence on serotonin levels (Doll H, Brown S, Thurston A, Vessey M. Pyridoxine (vitamin B6) and the premenstrual syndrome: a randomized crossover trial. JR Coll Gen Pract 1989; 39:364-8; Kleijnen J, Ter Riet G, Knipschild P. vitamin B6 in the treatment of the premenstrual syndrome: a review. Br J Obstet Gynaecol 1990; 97:847-52). However, its separate supplementation has not been shown as conclusive evidence of efficacy. Although available studies demonstrate improvement in PMS symptoms by taking vitamin B6 compared to placebo, these studies have poor methodological quality, undermining a recommendation to justify their use (Wyatt KM, Dimmock PW, Jones PW, PM Shaughn O'Brien. Efficacy of vitamin B-6 in the treatment of premenstrual syndrome: systematic review. BMJ 1999; 318: 1375-81).

The prior art also presents calcium as an important nutritional supplement for the treatment or alleviation of symptoms of PMT, PMS and PMDD. In the late 1990s, studies demonstrated the menstrual periodicity of hormones on the regulation of calcium metabolism, characterized by progressively increasing levels of parathyroid hormone (PTH) during the follicular phase, peaking in mid-cycle, with symptoms of PMS. (Thys-Jacobs S, Starkey P, Bernstein D, Tian J. Calcium carbonate and the premenstrual syndrome: effects on premenstrual and menstrual symptoms. Premenstrual Syndrome Study Group. Am J Obstet Gynecol 1998; 179:444-52). Changes in the concentrations of extracellular calcium could provide stimulatory effects on the neuromuscular junction, with states of agitation, mania and irritability often being reported in association with hypocalcaemia. A randomized clinical trial analyzing the use of calcium carbonate (dose of 1.0 g / day) for women with an average age of 21 years (SD = ± 3.6 years) and a diagnosis of PMS, noted that after an elapsed time of three months, the symptoms of fatigue and changes in appetite, accessed via questionnaire, showed significant improvement through the use of calcium carbonate (36% versus 52.3% and 52.7% versus 75% for the use of calcium and placebo, respectively) (Ghanbari Z, Haghollahi F, Shariat M, Foroshani AR Ashrafi M. Effects of calcium supplement therapy in women with premenstrual syndrome. Taiwan J Obstet Gynecol 2009; 48: 124-9). However, for complaints of headache, anxiety, agitation and irritability, significant changes were not observed.

Some studies correlating the use of magnesium and premenstrual symptoms have suggested that supplementation on the 15th day after onset of menses is an effective treatment for relieving symptoms of PMS (Facchinetti F, Borella P, Sances G, Fioroni L, Nappi RE, Genazzani AR. Oral magnesium successfully relieves premenstrual mood changes. Obstet Gynecol 1991; 78: 177-81; Walker AF, De Souza MC, Vickers MF, Abeyasekera S, Collins ML, Trinca LA. Magnesium supplementation alleviates premenstrual symptoms of fluid retention. J Womens Health 1998; 7:1157-65; De Souza MC, Walker AF, Robinson PA, Bolland K. A synergistic effect of a daily supplement for 1 month of 200 mg magnesium plus 50 mg vitamin B6 for the relief of anxiety related premenstrual symptoms: a randomized, double-blind, crossover study. J Womens Health Gend Based Med 2000; 9:131-9).

The prior art presents many patent documents and scientific articles which provide multivitamin compositions for the treatment of the symptoms of premenstrual tension. The application of the U.S. patent filed under the number US2013005679 provides compositions comprising vitamins B1, B2, B5, B6 and B12, folic acid, magnesium glycinate, and selenium. Despite having formulations which contain vitamins and minerals, these compositions do not contain carrier oils for the vitamins which are fat-soluble substances, which contribute to the reduction of bioavailability of such hydrophobic substances which are beneficial in the treatment of PMS symptoms. Additionally, the compositions of said document are administered for normalization of the menstrual cycle and for treating sleep disorders. The U.S. patent application US2008160083 provides a composition for the treatment of premenstrual symptoms. Said composition contains Chasteberry extract, pyridoxine, and magnesium. Said composition has no other vitamins, extracts or calcium, and does not provide a carrier for fat-soluble substances such as vitamins. Document WO2004004638 presents methods and medications for the treatment or improvement of certain inflammatory symptoms related to premenstrual syndrome, perimenopause, menopause, endometriosis, postpartum depression, or administration of hormonal contraceptives. The medications pleaded herein contain tocopherol, polyunsaturated omega-3 fatty acid (docosahexaenoic acid (DHA) or polyunsaturated fatty acid omega-9), a flavonoid and a mineral. The French document FR2806262 presents a nutritional supplement for treatment of premenstrual symptoms, menopausal problems, osteoporosis and cancer, containing evening primrose and borage oil, rich in fatty acids. Document US5569459 provides nutraceutical compositions for controlling premenstrual syndrome and alleviation of menopausal symptoms. The compositions of this document include phytoestrogens (isoflavones), licorice root extract, a sedative such as valerian root extract, passion fruit or ginseng, beta carotene, pyridoxine, vitamin E, calcium, magnesium, zinc, pantothenic acid and biologically acceptable vehicles. However, a disadvantage of the composition pled in this claim is that isoflavones may be harmful for women with a history of cancer.

There are already some drugs described in the prior art, such as Serafem (fluoxetine), the first drug approved by the Food and Drug Administration (FDA) for treating PMDD, NSAIDs such as ibuprofen and aspirin that assist in swelling and pain, and beta-blockers for migraine and anxiety. There are also alternative treatments such as lifestyle changes, healthy diet, exercise, stress relief therapies and even aroma therapy. Certain vitamins and supplements are also useful, such as B6, calcium, magnesium and vitamin E. Useful herbs include Vitex sp., Black cohosh, valerian, kava kava and Hypericum perforatum, commonly known as St. John's wort (Dennehy C, Tsourounis C. Maturitas." A review of select vitamins and minerals used by postmenopausal women." August 2010, 66 (4) :370-80 doi: 10.1016 / j.maturitas.2010.06.003. Epub June 26, 2010. Review; Nielsen FH, Milne DB, Gallagher S, Johnson L, Hoverson B. "Moderate magnesium deprivation results in calcium retention and altered potassium and phosphorus excretion by postmenopausal women." Magnes Res March 2007 20 (1):19-31; Nielsen FH, Milne DB, Klevay LM, Gallagher S, Johnson L." Dietary magnesium deficiency induces heart rhythm changes, impairs glucose tolerance, and decreases serum cholesterol in postmenopausal women." J Am Coll Nutr. April 2007, 26 (2):121-32; Wiacek M, Zubrzycki IZ, Bojke O, Kim HJ. "Menopause and age-driven changes in blood level of fat-and water-soluble vitamins." Climacteric. December 10, 2012; Aidelsburger P, Schauer S, K Grabein, J. Wasem "Alternative methods for the treatment of post-menopausal troubles." GMS Health Technol Assess. 2012 8: Doc03. doi: 10.3205/hta000101. Epub 2012 May; Hamidi MS, Corey PN, Cheung AM. "Effects of vitamin E on bone turnover markers among postmenopausal U.S. women." J Bone Miner Res June 2012 27 (6):1368-80. doi: 10.1002/jbmr.1566; Chai W, Conroy SM, Maskarinec G, Franke AA, Pagano IS, Cooney RV. "Associations between obesity and serum lipid-soluble micronutrients among premenopausal women." Nutr Res April 2010, 30 (4):227-32. doi: 10.1016 / j.nutres.2010.04.006; Ziaei S, Kazemnejad A Zareai M. "The effect of vitamin E on hot flashes in menopausal women." Gynecol Obstet Invest. 2007, 64 (4):204-7. Epub July 30, 2007; Carr BR, Khan N, Adams-Huet B, N Kakarla, Havelock JC, J. Gell "Effect of vitamin E supplementation with and without hormone therapy on circulatory inflammatory markers in postmenopausal women." Fertil Steril. March 2006, 85 (3):667-73; DG Carroll. "Non hormonal therapies for hot flashes in menopause." Am Fam Physician. February 1, 2006, 73 (3):457-64.Review; Rasool AH, Rehman A, WN Wan Yusuf, AR Rahman. "Vitamin E and its effect on arterial stiffness in postmenopausal women - a randomized controlled trial." Int J Clin Pharmacol Ther. December 2003, 41 (12) :587-92; Halbreich U, December 2004. "The diagnosis of premenstrual syndromes and premenstrual dysphoric disorder - clinical procedures and research perspectives." Gynecol. Endocrinol. 19 (6): p. 320-34; Endicott J, McLaughlin TP, AN Grudzinski, December 2003."Comparison of managed care charges among patients treated with selective serotonin reuptake inhibitors for premenstrual dysphoric disorder." J Clin Psychiatry 64 (12): p. 1511-6).

Some patent documents present multivitamin compositions, but for uses other than the relief of symptoms of premenstrual syndrome or hormonal disorders. Most of the compositions are indicated as a nutritional supplement. Patent document WO2012145369 provides a nutritional supplement containing coenzyme Q10, fish oil and vitamin D. In addition, the supplement may contain other substances such as evening primrose oil, resveratrol, vitamins B6, B12, and E, folic acid and piperine. Despite providing some substances of the present invention, the supplement of the referred document is administered with the aim of treating certain nutrient deficiencies, and improves cardiovascular disorders in individuals undergoing treatment with statins to decrease cholesterol levels. There is no description of use for relief of symptoms of premenstrual tension, hormonal disturbances or any related use. Document US7597909 presents an improved dietary supplement containing an electrolyte additive, such as calcium, magnesium or potassium, glucosamine sulfate, methylsulfonylmethane, Boswellia extract, phenylalanine, ascorbic acid and extracts of ginseng and ginkgo biloba. Document US2007292493, published on December 20, 2007, shows a nutraceutical composition and method for treating calcium deficiency. The composition consists of minerals such as magnesium, zinc, selenium, manganese or chromium; (2) a vitamin (vitamin A, vitamin D, vitamin C, vitamin E or B, choline, lecithin, inositol, PABA, biotin, or bioflavonoids, (3) a carotenoid (lycopene and lutein), (4) a hormone (dehydroepiandrosterone, progesterone, pregnenolone, or melatonin), (5) an amino acid (arginine, glutamine, lysine, phenylalanine, tyrosine, GABA, tryptophan, carnitine or acetyl L-carnitine), (6) a fatty acid such as fish oil or linseed oil, (7) a nutrient such as coenzyme Q10, (8) a stimulator of cartilage such as glucosamine, chondroitin, or MSM, (9) and a plant (ginkgo biloba, echinacea, or St. John's wort).

Some patent documents, such as CN102511567, NZ585280, US7875291, CN101336943, disclose compositions for reducing serum cholesterol and triglyceride levels to prevent the formation of thrombi. Most of these compositions are characterized by containing oils such as canola oil, soybean oil, sunflower oil, corn oil, safflower oil, linseed oil or evening primrose oil and some additional vitamins.

Other documents found in the prior art, containing vitamins and minerals are for cosmetic purposes. Document U.S. 2009/0269419 refers to a composition comprising vegetable oils rich in omega-3 or omega-6, at least one mineral, a trace element, vitamin C or derivative thereof, and at least one essential oil with anti-free radical properties for cell renewal, collagen protection and to combat pigmentation spots. Document U.S. 2008/0292613 refers to the use of a combination of B-complex vitamins, at least one essential oil comprising omega-3 and / or omega-6 and ah appropriate excipient for oral administration in the treatment of excess weight and resultant loosening of the skin.

Some articles and patent documents cite the effect of supplemental magnesium (as magnesium oxide - MgO) on the severity of premenstrual symptoms. According to studies, a daily supplement of 200 mg of magnesium (as MgO) reduced mild premenstrual symptoms (Ann F. et al. Journal of Women's Health. November of 1998, 7 (9): 1157-1165; US2013005679, US2008160083, document US5569459, among others). Some studies have identified the use of herbs, vitamins and minerals in the treatment of PMS. Some of the beneficial substances include calcium, vitamin B6, magnesium, ginkgo biloba, pyrrolidone, saffron, St. John's Wort, soy and vitamin E (AM Whelan et al. Herbs, vitamins and minerals in the treatment of premenstrual syndrome: a systematic review. The Canadian Journal of Clinical Pharmacology = Journal Canadien de Pharmacologie Clinique, 2009, 16 (3): 407-429).

The prior art also shows that the pathophysiology of PMS may include an interaction of ovarian hormones on brain neurotransmitters, such as serotonin and -aminobutyric acid (GABA) Backstrom et al. The role of hormones and hormonal treatments in premenstrual syndrome. CNS Drugs 2003; 17:325-42; Halbreich U. The etiology, biology, and evolving pathology of premenstrual syndromes. Psychoneuroendocrinology 2003, 28 (suppl 3:55-99). Thus, the B vitamins thiamine, niacin, riboflavin, vitamin B6, folic acid and vitamin B12 are involved in the metabolism of neurotransmitters, through different mechanisms. Riboflavin is needed to activate vitamin B6, which is a cofactor in the generation of serotonin from the amino acid tryptophan. Vitamin B6, vitamin B12 and folfolate are associated with the formation of S-adenosyl-methionine and tetraidrobiopterine, both required for the metabolism of serotonin and dopamine (RF Frankenburg. The role of one-carbon metabolism in schizophrenia and depression. Harv Rev Psychiatry 2007; 15: 146; Miller AL. The methylation, neurotransmitter, and antioxidant connections between folate and depression. Altern Med Show 2008; 13: 216).

Previous studies on the relationship between vitamin B-complex and PMS have generally been limited to randomized clinical trials with the efficacy of vitamin B6 in different doses as a treatment for premenstrual symptoms (KM Wyatt et al." Efficacy of vitamin B-6 in the treatment of premenstrual syndrome: systematic review." BMJ 1999; 318:1375-81; I Hagen et al." The effect of vitamin B-6 against premenstrual tension. A controlled clinical study." Acta Obstet Gynecol Scand 1985; 64:667-70). Some existing studies in the art have demonstrated that supplementation of vitamin B6 may reduce the occurrence and severity of symptoms (Kashanian M. et al. "Pyridoxine (vitamin B6) therapy for premenstrual syndrome." Int J Gynaecol Obstet, 2007, 96:43-4; P. Sharma et al. "Role of bromocriptine and pyridoxine in premenstrual tension syndrome." Indian J Physiol Pharmacol, 2007, 51:368-74; Williams MJ. Et al. "Controlled trial of pyridoxine in the premenstrual syndrome." J Int Med Res 1985 13:174-9).

The prior art shows several formulations for treating or alleviating the symptoms of premenstrual syndrome (PMS), premenstrual dysphoric disorder (PMDD), pre-menopausal, menopausal or female hormonal disorders. However, none of the documents found provides innovative formulations containing the ingredients shown in the present invention.

### SUMMARY OF THE INVENTION

The present invention relates to the field of nutraceuticals and drugs indicated for the treatment, prevention or alleviation of the symptoms of premenstrual syndrome (PMS), premenstrual dysphoric disorder (PMDD), premenopausal, menopausal or female hormonal disorders, in particular to compositions in the form of soft capsules containing vitamins, minerals and oils.

An embodiment of the invention provides compositions containing vitamins and minerals contained in compatible sources for association with a suitable vegetable oil. More preferably, the composition of the invention is for the treatment or reduction of symptoms related to premenstrual syndrome (PMS), premenstrual dysphoric disorder (PMDD), premenopaus, menopausal or female hormonal disorders, and comprises: (i) 25-700 mg of magnesium calculated as magnesium oxide or other compatible source; (ii) 120-1500 mg of calcium calculated as calcium carbonate or other compatible source; (iii) from 2.50 to 805 mg of vitamin E (as racealfatocoferol acetate or vitamin E 50% or other compatible source); ((iv) 0.32 to 200 mg of vitamin B6 calculated as pyridoxine hydrochloride or other compatible source and (v) 4 to 500 mg of gamma-linolenic acid, preferably from Borage or evening primrose oil, or oils from plants belonging to the Aceraceae, Boraginaceae, Cannabinaceae, Liliaceae, Onagraceae, Ranunculacea, Saxifragaceae, and Scrophulariaceae families.

A second embodiment relates to a pharmaceutical form containing the composition of the invention embodied as a soft capsule containing a liquid oily core and a shell suitable for enclosing a liquid formulation. Thus, this embodiment of the present invention enables the prescribing of a single product in the form of soft capsule containing all active and non-active ingredients which together increase bioavailability and hence the efficacy of the composition of the invention.

In a third embodiment of the invention, a process is provided for producing a soft capsule enclosing the composition of the invention, said process comprising the steps of: (a) feeding the oil(s) into a tank; (b) agitating said oil(s) at slow speed for a period of time sufficient to allow homogenization of the oily medium; (c) adding vitamins to the tank, maintaining the agitation for a period of time sufficient to allow dissolution in the oily medium; (d) adding mineral ingredients by increasing the stirrer speed to a mean value, maintaining this stirring for a period of time sufficient to maintain the mineral particles in suspension for encapsulation; and (e) complete the composition btained in step (d) in a wrapping material to form a soft capsule.

A fourth embodiment relates to the use of the composition of the present invention in the manufacture of a product for the treatment, prevention or alleviation of the symptoms of premenstrual syndrome (PMS), premenstrual dysphoric disorder (PMDD), premenopausal, menopausal, or female hormonal disorders.

These and other objects of the present invention will be better understood and appreciated from the following detailed description of the invention and the attached claims.

### BRIEF DESCRIPTION OF THE FIGURES

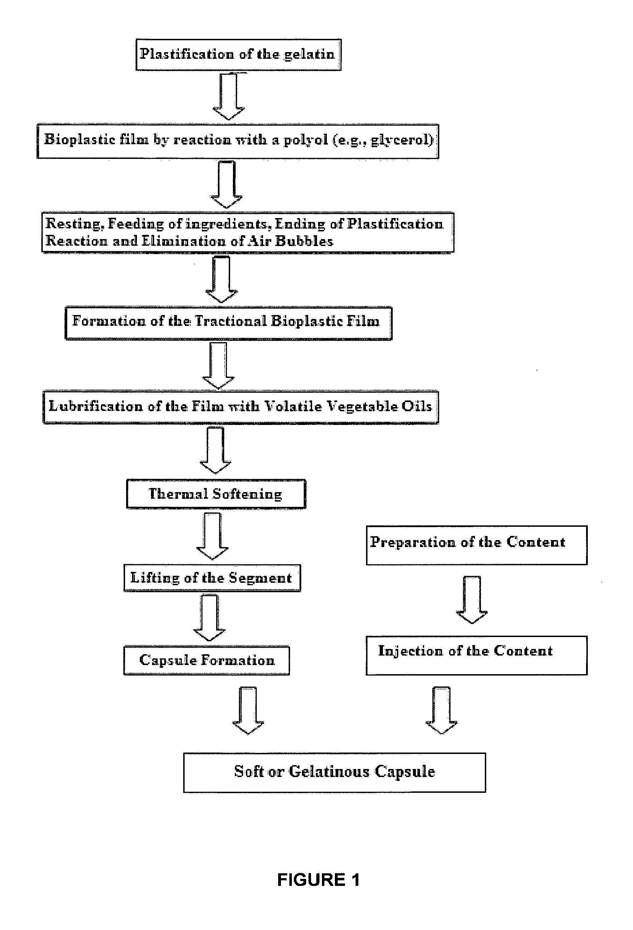

Figure 1 represents the process of obtaining a soft or gelatinous capsule which is based on the formation of the capsule (shell) and in preparing the contents through solubilization and incorporation of vitamins and minerals in the oil medium following encapsulation. The process is best detailed below in the detailed description of the invention.

### DETAILED DESCRIPTION OF THE INVENTION

The present invention relates to the field of foods and medicines for the treatment or relief of premenstrual syndrome (PMS), premenstrual dysphoric disorder (PMDD), premenopausal, menopausal or female hormonal disorders; in particular the compositions in the form of a soft capsule, containing vitamins, minerals and oils.

The existing prior art presents vitamins and minerals in tablets separately of evening primrose or borage oil in a soft capsule. This complicates the prescription, because it requires the patient to ensure the purchase and the intake of two (2) products (tablet and softgel), which may reduce treatment compliance.

The compositions of the present invention contain magnesium which come from a compatible source selected from the group consisting of magnesium carbonate hydroxide, magnesium chloride, magnesium gluconate, magnesium glycerophosphate, magnesium hydroxide, magnesium lactate, magnesium oxide, magnesium hydrogen phosphate, tribasic magnesium phospate, magnesium sulfate, magnesium acetate, magnesium salts of citric acid and magnesium carbonate. (magnesium salts of citric acid = magnesium citrate)

The compositions of the present invention contain calcium derived from a compatible source selected from the group consisting of calcium carbonate, calcium chloride, calcium citrate, calcium gluconate, calcium glycerophosphate, calcium L-lactate, calcium hydroxide, calcium oxide, monobasic calcium phosphate, dibasic calcium phosphate, tribasic calcium phosphate and calcium sulfate.

The compositions of the present invention contain vitamin E in the form of a compound selected from the group consisting of D-alpha tocopherol, DL-alpha tocopherol, D-alpha-tocopheryl acetate, DL-alpha tocopheryl acetate (racealfatocoferol acetate) D-alpha-tocopheryl acid succinate, DL-alpha-tocopheryl succinate and DL-alpha-tocopheryl.

The compositions of this invention contain vitamin B6 in the form of a compound selected from the group consisting of pyridoxine hydrochloride and pyridoxal 5-phosphate.

Additionally, the compositions of this invention may contain other ingredients selected from the group consisting of folic acid, iron, zinc, beta carotene, thiamine, riboflavin, cholecalciferol, nicotinamide, ascorbic acid, biotin, pantothenic acid, chromium, copper, iodine, manganese, molybdenum and selenium.

One of the most important aspects of the present invention is that a lipophilic medium is provided, sufficient to ensure dissolution of the lipophilic vitamins and to maintain the mineral particles in the composition in suspension. This medium is rich in gamma-linolenic acid (GLA) oils derived from plants of the group consisting of *Oenothera biennis* (Evening Primrose), *Borago officinalis L.* (Borage), *Aceraceae, Boraginaceae, Cannabinaceae, Liliaceae, Onagraceae, Ranunculacea, Saxifragaceae, Scrophulariaceae.* Preferably, the oil used in the composition of the present invention is selected from the group consisting of borage oil, evening primrose oil, or mixtures thereof.

In a preferred embodiment, the compositions of the present invention include magnesium (as magnesium oxide), calcium (as calcium carbonate), vitamin E (such as racealfatocoferol acetate or vitamin E 50%), vitamin B6 (as pyridoxine hydrochloride) or other sources of vitamins and minerals compatible with the technology, and borage oil and / or evening primrose oil as well as other oils with GLA belonging to the families Aceraceae, Boraginaceae, Cannabinaceae, Liliaceae, Onagraceae, Ranunculacea, Saxifragaceae and Scrophulariaceae.

The present invention solves the problem of treatment compliance which exists in the prior art because it provides a formulation containing vitamins and minerals from compatible sources for association with borage oil or evening primrose oil, or a mixture of both oils, and other oils with GLA belonging to the families Aceraceae, Boraginaceae, Cannabinaceae, Liliaceae, Onagraceae, Ranunculacea, Saxifragaceae and Scrophulariaceae, in the form of a soft capsule containing a liquid oil core in which the suspended vitamin and mineral ingredients are dissolved. The feasibility of the soft capsule leads to ease in prescribing a single product.

It is understood by soft gel capsules, or simply soft capsules, are those formed by a hermetically sealed flexible film called a shell or mantle, having in its interior a liquid material content called a load, mass or filling. Soft capsules are used for the primary packaging of non-aqueous substances, in the packaging of food, nutraceuticals, pharmaceuticals and cosmetics.

The process for obtaining soft capsules of the present invention follows the classic method of obtaining soft capsules. An aqueous gelatin mass formulated with particular gelatin, plasticizers, colorants, preservatives and other substances that will form the shell of the capsules is produced in a special-reactor. This gelatin is maintained as a fluid gel in thermostatic containers in the temperature range of 50° C. This gel is spread out by means of devices called distribution boxes or pits, on the surface of two independent chilled cylinders that rotate at very slow speed. The thickness of the gel layer deposited on the surface of the cylinder is controlled by adjusting the pit, and will determine the thickness of the dried shell in the final product. The layer of warm gel in contact with the cold surface of the cylinder acquires mechanical resistance and forms a film with bioplastic characteristics. The film formed in each cylinder is tractioned by a set of rotating shafts which receives a layer of volatile vegetable oil on each of its sides, allowing it to follow the surface of the forming arrays. These cylindrical arrays are two in number, and rotate in opposite directions, compressing the two films in their internal cavities while simultaneously promoting the cutting of capsules according to the geometry provided in their format. A device called a Teflon wedge is provided between the two rotating dies. This wedge is heated by electrical resistance and has internal holes where the product is injected into the wells. A pump working synchronously with the matrices measures and injects the content at the exact moment of sealing. The capsules thus formed are detached from the rest of the film and go to a collecting conveyor, which follow to the later stages of drying. The remaining cutfilm of the capsules is called net and may return to the beginning of the process and be incorporated into the new preparations of gelatin. The capsules pass through a freeze drying process of circulating dehumidified air. When dried, the capsules acquire their final form and become the product we know.

The objective of the plasticized gelatin is to obtain a bioplastic film by reaction with a polyol, usually glycerol. This step is performed under a vacuum in reactors constructed of 316-L stainless steel to eliminate air bubbles.

Following this, the step of resting and feeding is performed to finalize the reaction of the plasticizers and the elimination of air bubbles resulting from discharge from the reactor. This step is carried out in thermostatic stainless steel containers. Next, transfer to the machine is performed, always warm, for feeding of the equipment, which is performed by gravity, peristaltic pumps, lobe pumps, pressurization, etc. The distribution and spreading of the warm gelatin is made in the machine thermostatic box by mechanical feeders driven by a variable speed step motor. The formation of tractional bioplastic film is performed on the cooled molding roller.

Lubrication of the film, which is performed in the film lubricator cylinders, is powered by pneumatic injection and made with essential oils in order to prevent adhesions in later steps.

Next, the thermal softening is performed in the Teflon segment thermostat with the aim of preparing the film for pressing in the forming matrix. Lifting of the segment, by pneumatic control, is performed in order to remove it from the matrix during changes and adjustments to machine settings.

Finally, capsule formation is performed in the forming dies according to the desired dimensions, shapes and weights. The preparation of content is carried out in reactors, mixers, filters, blenders and other equipment suitable for product pharmacotechnique. The dosage and injection of the content are performed by the metering pump. Figure 1 shows the schematic process of the invention.

Next, the following steps are performed: sealing of the capsule, removal of the obtained capsules, cleaning up of resulting scraps, transporting of the capsule, removal of the volatile vegetable oil lubricant, preparation for final drying, final drying and completion of the industrial production process.

As detailed in the background of the invention, vitamin B6 acts as a coenzyme in the biosynthesis of dopamine and serotonin (neurotransmitters possibly involved in the etiology of PMS). Its administration correlates with an increase in serum progesterone in the mid-luteal period with an influence on serotonin levels (Doll H, Brown S, Thurston A, Vessey M. "Pyridoxine (vitamin B6) and the premenstrual syndrome: a randomized crossover trial." JR Coll Gen Pract 1989; 39:364-8; Kleijnen J, Ter Riet G, Knipschild P. "Vitamin B6 in the treatment of premenstrual syndrome: a review." Br J Obstet Gynaecol 1990, 97:847-52). Vitamin E, also known as tocopherol, also contributes to the improvement of the symptoms of PMS. Vitamin E is a fat-soluble nutrient , i.e., it is stored in fat cells and released in small doses to meet the body's needs.

Calcium is an important nutritional supplement for the relief or treatment of PMS symptoms, PMT and PMDD. Calcium can have stimulatory effects on the neuromuscular junction, and may also improve symptoms of fatigue and changes in appetite.

Magnesium is involved in key biological processes in the body, among them, it acts as part of normal cardiovascular function, muscle function, nervous system function and the formation and maintenance of bones. Other health benefits for women with the use of magnesium include the relief of menopausal symptoms and premenstrual syndrome (PMS), protein synthesis and improving the function of the parathyroid (Facchinetti F, Borella P, Sances G, Fioroni L, Nappi RE, Genazzani AR. "Oral magnesium successfully relieves premenstrual mood changes. "Obstet Gynecol 1991, 78:177-81; Walker AF, De Souza MC, Vickers MF, Abeyasekera S, Collins ML, Trinca LA. "Magnesium supplementation alleviates premenstrual symptoms of fluid retention." J Womens Health 1998 ,7:1157-65; De Souza MC, Walker AF, Robinson PA, Bolland K." The synergistic effect of a daily supplement for 1 month of 200 mg magnesium plus 50 mg vitamin B6 for the relief of anxiety related premenstrual symptoms: a randomized, double-blind, crossover study." J Womens Health Gend Based Med 2000,9:131-9).

Evening primrose oil is obtained from the seeds of the Oenothera biennis plant species. It is an oil rich in essential fatty acid of the omega-6 family, such as linolenic acid (LA) and gamma linolenic acid (GLA).

Borage oil, which can also be used in the present invention is shown to assist in hormonal regulation (PMS, menstrual pain and climacteric). It is a natural source of gamma linolenic acid, which is a polyunsaturated fatty acid. It is also a precursor of prostaglandins, which can aid in lowering blood pressure.

Finally, other oils containing GLA belonging to the families Aceraceae, Boraginaceae, Cannabinaceae, Liliaceae, Onagraceae, Ranunculacea, Saxifragaceae and Scrophulariaceae can be used.

The compositions of the present invention will be better understood from the following examples. They will be presented, as non-limiting examples, as compositions in the form of a soft capsule containing vitamins and minerals with compatible sources to associate with oil (evening primrose oil or borage oil, or a mixture of both oils, and other oils containing GLA also belonging to the families Aceraceae, Boraginaceae, Cannabinaceae, Liliaceae, Onagraceae, Ranunculacea, Saxifragaceae and Scrophulariaceae).

### Examples

8 formulations were prepared according to the invention as shown in Table 1.

**Table 1: Compositions according to the present invention**

| **Composition/ Ingredient** | **Magnesium (mg)** | **Calcium (mg)** | **Vitamin E (mg)** | **Vitamin B6 (mg)** | **Borrage Oil (mg)** | **Prímrose Oil (mg)** |
|---|---|---|---|---|---|---|
| 1 | 260 | 250 | 10 | 1.3 | 800 | - |
| 2 | 260 | 250 | 10 | 1.3 | 20 | - |
| 3 | 260 | 250 | 10 | 1.3 | 650 | 350 |
| 4 | 260 | 250 | 10 | 1.3 | - | 2000 |
| 5 | 65 | 250 | 10 | 4.0 | - | 2000 |
| 6 | 65 | 250 | 2,5 | 0.325 | - | 2000 |
| 7 | 700 | 1500 | 30 | 200 | - | 2000 |
| 8 | 65 | 250 | 600 | 0.325 | 800 | - |

As previously defined herein, the amounts of the ingredients listed in Table 1 have the following meanings:
- Magnesium employed as magnesium oxide or coming from another compatible source;
- Calcium employed as calcium carbonate or coming from another compatible source;
- Vitamin E used as racealfatocoferol acetate (vitamin E 50%) or coming from another compatible source;
- Vitamin B6 employed as pyridoxine hydrochloride or coming from another compatible source;
- Borage oil (oil extracted from the seed of the plant Borago officinalis L.) (containing 23% GLA) or another oil with GLA (gamma linolenic acid);
- Evening primrose oil (containing 9% GLA) or another oil with GLA.

The compositions of the present invention are prepared as follows:
Step 1 - Weigh all the ingredients as indicated in the weighing record;
Step 2 - Position the oil(s) in the tank and stir with slow speed for a period of 1 to 60 minutes, preferably for a period of 3 to 10 minutes, more preferably for a period of 5 minutes;
Step 3 - Under medium agitation, add the vitamins and continue stirring for a period of 1 to 60 minutes, preferably for a period of 3 to 10 minutes, more preferably for a period of 5 minutes;
Step 4 - Under medium agitation, add the mineral ingredients and continue stirring for a period of 1 to 60 minutes, preferably for a period of 3 to 10 minutes, more preferably for a period of 5 minutes;
Step 5 - Remove the Quality Control test sample.
Step 6 - After approval of Quality Control, release for bottling.

## Claims

1. Composition for the treatment or reduction of symptoms related to premenstrual syndrome (PMS), premenstrual dysphoric disorder (PMDD), premenopause, menopause and female hormonal disorders, wherein comprising:
(i) 25-700 mg of magnesium calculated as magnesium oxide or other compatible source;
(ii) 120-1500 mg of calcium calculated as calcium carbonate or other compatible source;
(iii) 2.50 to 805 mg of vitamin E (as racealfatocoferol acetate or Vitamin E 50% or other compatible source);
(iv) 0.32 to 200 mg of Vitamin B6 calculated as pyridoxine hydrochloride or other compatible source;
(v) 4-500 mg of gamma-linolenic acid from borage or evening primrose oil and other oils or mixtures of oils with GLA belonging to the Aceraceae, Boraginaceae, Cannabinaceae, Liliaceae, Onagraceae, Ranunculacea, Saxifragaceae and Scrophulariaceae family.

2. Composition according to claim 1, **wherein** said gamma-linolenic acid is derived from borage oil, evening primrose oil, or mixtures thereof.

3. Composition according to claim 1, **which contains** a compatible source of magnesium, which is selected from the group consisting of magnesium hydroxide carbonate, magnesium chloride, magnesium gluconate, magnesium glycerophosphate, magnesium hydroxide, magnesium lactate, magnesium oxide, magnesium hydrogen phosphate, tribasic magnesium phosphate, magnesium sulfate, magnesium acetate, magnesium citrate salts and magnesium carbonate salts.

4. Composition according to claim 1, **which contains** a compatible source of calcium from a selected group consisting of calcium carbonate, calcium chloride, calcium citrate, calcium gluconate, calcium glycerophosphate, calcium L-lactate, calcium hydroxide, calcium oxide, monobasic calcium phosphate, dibasic calcium phosphate, tribasic calcium phosphate and calcium sulfate.

5. Composition according to claim 1, **which contains** a compatible source of vitamin E from a selected group consisting of d-alpha-tocopherol, DL-alpha-tocopherol, acetate D-alpha-tocopheryl, acetate DL-alpha-tocopheryl, D-alpha-tocopheryl succinate acid, DL-alpha-tocopheryl succinate acid, DL-alpha-tocopheryl succinate of DL-alpha-tocopheryl.

6. Composition according to claim 1, **which contains** a compatible source of vitamin B6 selected from the group consisting of pyridoxine hydrochloride and pyridoxal 5-phosphate.

7. Composition according to claim 1, **which comprises** other ingredients selected from the group consisting of folic acid, iron, zinc, beta carotene, thiamine, riboflavin, cholecalciferol, nicotinamide, ascorbic acid, biotin, pantothenic acid, chromium, copper, iodine, manganese, molybdenum and selenium.

8. Composition according to claim 1, **which comprises** minerals having mineral chelates as their source.

9. Pharmaceutical form for the treatment or reduction of symptoms related to premenstrual syndrome (PMS), premenstrual dysphoric disorder (PMDD), premenopause, menopause and female hormonal disorder, **wherein** is in a soft capsule form and comprises: (i) an oily liquid core containing the composition as defined in any one of claims 1 to 8; and (ii) a solid gelatin shell forming a soft capsule.

10. Pharmaceutical form according to claim 9, **wherein** said oily core comprises vitamins dissolved in the lipophilic medium and suspended mineral ingredients in the same medium.

11. Process for producing a pharmaceutical form as defined in claims 9 and 10 enclosing the composition as defined in claims 1 to 8, wherein said method comprising the steps of:
(a) supplying of oil(s) to a tank;
(b) agitating said oil(s) at slow speed for a period of time sufficient to allow homogenization of the oily medium;
(c) adding vitamins to the tank, maintaining agitation for a period sufficient to permit dissolution in the oily medium;
(d) adding mineral ingredients by increasing the stirrer speed to a mean value, maintaining this stirring for a period of time sufficient to maintain the mineral particles in suspension for encapsulation; and
(e) completing the composition obtained in step (d) in a shell of material forming a soft capsule.

12. Process according to claim 11, **wherein** said time period of steps (b) and (c) is in the range of 1 to 60 minutes.

13. Process according to claim 11, **wherein** said time period of step (d) is in the range of 1 to 60 minutes.

14. Process according to claim 13, **wherein** said time period of steps (b) and (c) is in the range of 3 to 10 minutes.

15. Process according to claim 14, **wherein** said time period of steps (b) and (c) is 5 minutes.

16. Process according to claim 11, **wherein** said increased agitation speed of step (d) is a speed in the range 1 to 60 minutes.

17. Process according to claim 16, **wherein** said increased agitation speed of step (d) is a speed in the range 3 to 10 minutes.

18. Process according to claim 17, **wherein** said increased agitation speed of step (d) is 5 minutes.

19. Use of the composition defined in any one of claims 1 to 8, **wherein** it is the manufacture of a product for the treatment, prevention or alleviation of the symptoms of premenstrual syndrome (PMS), premenstrual dysphoric disorder (PMDD), premenopause, menopause and female hormonal disorders.

20. Use according to claim 19, **wherein** said composition is in the form of a soft capsule.
